Europäisches Patentamt

European Patent Office

Office européen des brevets

(11) Numéro de publication: **0 183 593**
**B1**

# (12) FASCICULE DE BREVET EUROPEEN

(45) Date de publication du fascicule du brevet:
**10.01.90**

(21) Numéro de dépôt: **85402160.7**

(22) Date de dépôt: **08.11.85**

(51) Int. Cl.⁴: **A 61 M 16/00, G 01 L 19/12**

(54) **Appareil respiratoire.**

(30) Priorité: **13.11.84 FR 8417236**

(43) Date de publication de la demande:
**04.06.86 Bulletin 86/23**

(45) Mention de la délivrance du brevet:
**10.01.90 Bulletin 90/2**

(84) Etats contractants désignés:
**AT BE CH DE FR GB IT LI LU NL SE**

(56) Documents cités:
**FR-A- 2 346 025
GB-A- 2 004 180
GB-A- 2 089 497
US-A- 3 831 596**

(73) Titulaire: **L'AIR LIQUIDE, SOCIETE ANONYME POUR L'ETUDE ET L'EXPLOITATION DES PROCEDES GEORGES CLAUDE, 75, Quai d'Orsay, F-75321 Paris Cédex 07 (FR)**

(72) Inventeur: **Champain, Roger, 2, rue Ferme de l'Hopital, F-78350 Les Loges-en-Josas (FR)**

(74) Mandataire: **Vesin, Jacques et al, L'AIR LIQUIDE, SOCIETE ANONYME POUR L'ETUDE ET L'EXPLOITATION DES PROCEDES GEORGES CLAUDE 75, quai d'Orsay, F-75321 Paris Cédex 07 (FR)**

## Description

La présente invention concerne un appareil pour le traitement des déficiences respiratoires d'un patient par la fourniture d'un fluide de respiration à partir d'une source de fluide de respiration afin de compléter la fourniture d'oxygène inspiré par le patient dans l'air ambiant, cet appareil comportant un premier conduit reliant la source de fluide de respiration à la bouche ou aux narines du patient, une vanne dont l'ouverture et la fermeture peuvent être commandées électriquement étant placée dans ledit premier conduit de manière à contrôler le débit de fluide de respiration délivré au patient et des moyens pour commander l'ouverture ou la fermeture de la vanne en liaison avec les phases d'inspiration et d'expiration du patient.

L'invention concerne également un dispositif de commande utilisable dans un tel appareil.

On connaît déjà divers appareils pour le traitement des déficiences respiratoires d'un patient par apport d'oxygène additionnel délivré, à partir d'une source, par un conduit débouchant au voisinage des voies respiratoires du patient. L'un de ces appareils le plus répandu est la lunette d'oxygénothérapie qui peut s'adapter sur le visage et qui comporte deux appendices permettant un apport d'oxygène aux deux narines du patient.

Ces appareils sont généralement pourvus d'une simple vanne à commande manuelle insérée dans le conduit d'alimentation de l'oxygène et que le patient ouvre lorsque l'appareil est en service. De ce fait, les voies respiratoires du patient sont alimentées en oxygène en permanence, aussi bien pendant les phases inspiratoires que pendant les phases expiratoires, ce qui se traduit par un gaspillage d'oxygène.

Pour remédier à cet inconvénient, on a envisagé de réaliser des dispositifs basés sur la détection de la dépression nasale au cours de la phase inspiratoire, afin de ne commander l'alimentation en oxygène que pendant ces phases inspiratoires. Un appareil de ce genre qui est décrit dans la demande de brevet européen 99 283 est basé sur la mesures des différences de température entre l'air inspiré et l'air expirée.

Dans les pays tropicaux, par exemple, ou dans un milieu hospitalier à température élevée, la différence de température entre les gaz inspirés et expirés peut s'avérer insuffisante pour un fonctionnement fiable de l'appareil.

Par ailleurs, il est connu de GB-A 2 089 497 un appareil de contrôle de la pression d'un fluide délivré par une pompe à un utilisateur, dans lequel le fluide est injecté dans une chambre étanche d'un côté d'une membrane flexible. Cette membrane flexible est liée à une seconde membrane flexible solidaire d'un bras agissant sur un levier dont le mouvement vers le haut déclenche la coupure du faisceau du dispositif optoélectronique lorsqu'une surpression du fluide est détectée ce qui coupe l'injection de fluide. Le levier est maintenu en position basse par un ressort qui s'oppose au mouvement de la membrane flexible.

Un tel système présente plusieurs inconvénients: sa structure est compliquée, ce qui en fait un objet assez coûteux, tandis que sa sensibilité ne peut être adaptée aux faibles pressions différentielles qui sont présentes en oxygénothérapie.

Le problème particulier qui se pose dans ce domaine technique est en effet de réaliser des dispositifs qui soient sensibles à de très faibles différences de pression entre la pression atmosphérique et la pression d'inspiration ou d'expiration d'un malade qui peut être dans un grand état de faiblesse et auquel on ne peut demander d'importants efforts, l'injection de fluide de respiration ne devant être réalisée que pendant les phases d'inspiration du patient, de manière à économiser ledit fluide et permettre nottament la réalisation d'appareils portatifs ayant une autonomie suffisante malgré la faible capacité de la source de fluide de respiration transportée par le patient.

L'appareil de traitement selon l'invention est caractérisé en ce que les moyens pour commander l'ouverture ou la fermeture de la vanne sont constitués d'une chambre dans laquelle est placé un réservoir sous forme d'une baudruche souple, ce réservoir ayant une ouverture débouchant à l'air ambiant à travers une cloison de la chambre, d'un écran solidaire d'une paroi mobile dudit réservoir, d'un dispositif optoélectronique comportant un émetteur et un récepteur d'un faisceau lumineux, d'un circuit électronique de commande connecté, d'une part, au récepteur et, d'autre part, à la vanne, ainsi que d'un second conduit reliant la chambre à la bouche ou aux narines du patient, la paroi mobile dudit réservoir étant soumise d'un côté à la pression de l'air ambiant et de l'autre à la pression de l'air dans la chambre venant de/où allant vers le patient par l'intermédiaire du second conduit, la différence de pression de part et d'autre de la paroi mobile du réservoir engendrant un déplacement de l'écran en direction du gaz de plus faible pression, de sorte que l'écran vient couper au moins partiellement ou cesse de couper totalement le faisceau lumineux entre émetteur et récepteur, ledit récepteur engendrant des signaux de déclenchement du circuit électronique de commande qui provoque l'ouverture ou la fermeture de la vanne.

Dans cette forme de réalisation, le fonctionnement consiste à déplacer la paroi mobile vers le bas: le faisceau lumineux qui n'était pas interrompu lorsque la paroi mobile était au repos est interrompu lorsque la paroi mobile est déplacée vers le bas.

Selon une autre forme de réalisation de l'invention, l'appareil pour le traitement des déficiences respiratoires d'un patient est caractérisé en ce que les moyens pour commander l'ouverture ou la fermeture de la vanne sont constitués d'une chambre dans laquelle est placé un réservoir sous forme d'une baudruche souple, d'un écran solidaire d'une paroi mobile dudit réservoir, d'un dispositif optoélectronique comportant un émetteur et un récepteur d'un faisceau lumineux, d'un circuit électronique de commande connecté, d'une part, au récepteur et, d'autre part, à la vanne, ainsi que d'un second conduit reliant l'intérieur du réservoir au premier conduit en aval de la vanne, l'intérieur de la chambre étant mis à l'atmosphère au moyen d'un trou percé dans une des parois de cette chambre, de sorte que la paroi mobile dudit réservoir soit soumise du côté de la chambre à la pression de l'air ambiant et de l'autre à la pression de l'air conduit, la différence de pression de part et d'autre de la paroi mobile du réservoir engendrant un déplacement de l'écran en direction du gaz de plus faible pression, de sorte que l'écran vient couper au moins partiellement ou cesse de couper totalement le faisceau lumineux entre émetteur et récepteur, ledit récepteur engendrant des signaux de déclenchement du circuit électronique de commande qui provoquent l'ouverture ou la fermeture de la vanne.

Dans cette seconde forme de réalisation, la paroi mobile étant horizontale au repos, le fonctionnement consiste à déplacer vers le haut la paroi: le faisceau lumineux initialement interrompu par l'écran lorsque la paroi est horizontale ne l'est plus lorsque la paroi est déplacée vers le haut.

D'une façon générale, dans l'une et l'autre forme de réalisation, l'appareil peut déclencher l'ouverture de la vanne d'injection d'oxygène (ou d'un autre gaz ou fluide) soit à la fin de la phase d'expiration du patient, soit au début de la phase d'inspiration du patient.

Dans certains cas, il est possible d'utiliser un troisième mode de fonctionnement dans lequel le faisceau n'est pas interrompu lorsque la paroi mobile est déplacée vers le haut, mais est interrompu (partiellement ou totalement) seulement lorsque la paroi mobile est déplacé vers le bas.

Dans tous les cas, on ajustera la position de la lamelle par rapport au faisceau, au repos, soit à l'aide de moyens de réglage appropriés pour déplacer l'écran vers le haut ou vers le bas, soit en choisissant un écran de hauteur différente suivant le mode de fonctionnement souhaité. En pratique, on constate que la sensibilité des circuits électroniques reliant le récepteur à la vanne sont suffisants pour détecter avec un grande fiabilité une variation du flux lumineux capté par le récepteur. Ainsi est-il possible de régler la position de l'écran de telle sorte qu'il intercepte partiellement le faisceau au repos de la paroi mobile, et qu'il n'intercepte plus le faisceau en position haute (ou qu'il l'intercepte complètement en position basse) pour que cette différence d'intensité captée par le récepteur se traduise par l'émission d'un signal de commande fiable. Ceci permet d'augmenter encore la sensibilité du dispositif selon l'invention. Pour éviter toutefois, lorsque la sensibilité du dispositif devient trop grande, une instabilitée du système, engendrant de manière trop rapprochée des ordres d'ouverture et de fermeture de la vanne avant un intervalle de temps prédéterminé après l'ouverture (et/ou vice-versa).

On décrira ci-après, à titre d'exemples non limitatifs, diverses formes d'exécution de la présente invention, en référence aux dessins annexés sur lesquels:

la fig. 1 est un schéma synoptique d'un détecteur de faibles dépressions utilisé avec une lunette d'oxygénothérapie du commerce;

la fig. 2 est un schéma synoptique d'une variante d'exécution du détecteur de faible dépression;

la fig. 3 est un schéma synoptique d'un détecteur de dépression utilisable avec une lunette d'oxygénothérapie comportant un tuyau annexe de prise de pression;

la fig. 4 est un schéma électrique explicitant le déclenchement d'une électrovanne à l'aide du dispositif selon l'invention;

la fig. 5 est une variante de la fig. 2 avec une électrovanne trois voies;

la fig. 6 est un exemple d'un système avec déclenchement en fin d'expiration; et

la fig. 7 est une vue détaillée d'une réalisation d'un dispositif conforme à l'invention.

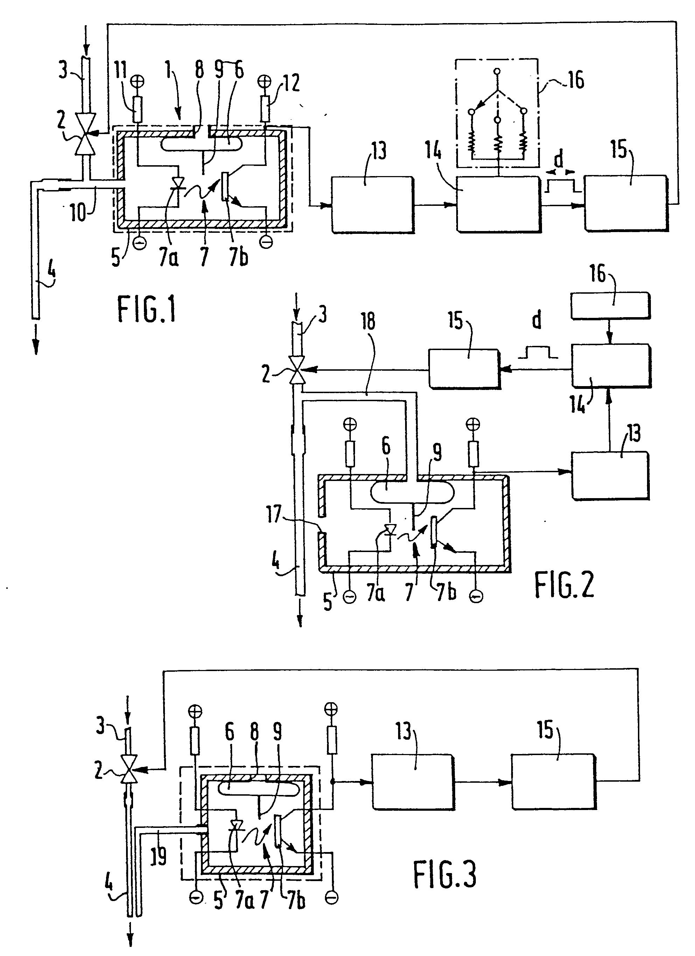

Le détecteur de dépression qui est représenté sur la fig. 1 et qui est désigné dans son ensemble par la référence 1, est destiné à piloter une électrovanne 2 branchée sur une canalisation 3 d'alimentation en oxygène laquelle est reliée à une lunette d'oxygénothérapie dont seule la partie extrême 4 est représentée sur le dessin.

Le détecteur de faible dépression 1 comporte une chambre étanche 5 à l'intérieur de laquelle sont logés, d'une part, une baudruche très sensible 6 et, d'autre part, un capteur optoélectronique 7. La baudruche 6 utilisée dans le détecteur suivant l'invention peut avoir, par exemple, un diamètre de 28,4 mm et une épaisseur de 0,2 mm et elle peut être en silicone, du type connu sous le nom VERSILIC 3706H fabriqué par la SOCIETE VERNEPET. La baudruche 6 prend appui sur une paroi de la chambre 5 et son volume interne communique avec l'extérieur par l'intermédiare d'un trou 8 percé dans cette paroi, si bien que la baudruche 6 est soumise, à l'intérieur, à la pression atmosphérique. Cette baudruche 6 porte par ailleurs, sur sa face frontale mobile, une lamelle 9 formant écran et qui est susceptible d'être interposée entre l'émetteur 7a (diode émettrice) et le récepteur 7b (phototransistor) du capteur optoélectronique 7. Par ailleurs, l'intérieur de la chambre étanche 5 communique avec la canalisation 3 d'alimentation en oxygène, en aval de la vanne 2, par l'intermédiaire d'un conduit 10.

La souplesse de la baudruche doit être telle qu'une variation de pression de 1 mm d'eau entraîne un déplacement de l'écran d'au moins 0,05 mm. En pratique, on préfère utiliser une baudruche de souplesse le plus élevée possible. On peut couramment obtenir dans le commence des baudruches dont la souplesse est de l'ordre de 0,15 mm par mm d'eau.

Le capteur optoélectronique 7 est relié à un montage électronique qui commande la vanne 2. Plus particulièrement, son émetteur 7a est relié à une source d'alimentation électrique, par l'intermédiaire d'une résistance 11. De la même façon, son récepteur 7b est également connecté à cette source d'alimentation électrique 11b, en série avec une résistance 12, et le signal de sortie du capteur optoélectronique, prélevé entre la résistance 12 et le collecteur du phototransistor 7b constituant le récepteur, est appliqué à un circuit de mise en forme 13. La sortie de ce circuit de mise en forme 13 est connectée à l'entrée d'un monostable 14 dont la sortie est à son tour reliée à un circuit 15 de commande de l'électrovanne 2. Un circuit 16 permettant de faire varier la durée d'enclenchement du monostable 14, c'est-à-dire la durée d de son signal de sortie, est prévu afin de permettre de choisir à volonté la durée de la phase d'inspiration de l'oxygène.

Le fonctionnement du détecteur qui vient d'être décrit est le suivant: tant que le patient n'inspire pas, la pression régnant dans la lunette d'oxygénothérapie 4, le conduit 10 et l'intérieur de la chambre 5 qui est due à l'expiration du patient, est relativement élevée, si bien que la baudruche 6 est aplatie et que l'écran 9 n'est pas interposé entre l'émetteur 7a et le récepteur 7b du capteur optoélectronique 7, ce qui est également le cas au repos. La vanne 2 est maintenue fermée et le patient n'est pas alimenté en oxygène. Au tout début d'une phase d'inspiration, il se produit, dans la lunette d'oxygénothérapie 4 et par conséquent à l'intérieur de la chambre étanche 5, une faible dépression qui provoque immédiatement un gonflement de la baudruche 6 si bien que la lamelle formant écran 9 vient s'interposer totalement ou en partie selon réglage entre l'émetteur 7a et le récepteur 7b du capteur optoélectronique 7. Ce capteur émet par conséquent, sur le collecteur du phototransistor 7b, une impulsion qui est mise en forme par le circuit 13 et qui est appliquée à l'entrée du monostable 14. Ce dernier émet à sa sortie une impulsion créneau de durée d qui peut être choisie à volonté au moyen du circuit de commande 15 qui provoque alors l'ouverture de la vanne 2 pendant une durée déterminée par le temps d'enclenchement du monostable 14. Le patient reçoit ainsi, lors de chaque phase inspiratoire, une quantité d'oxygène bien déterminée. A la fin de la durée d le monostable 14 revient au repos et provoque, par l'intermédiaire du circuit de commande 15, la fermeture de l'électrovanne 2 (cas d'une électrovanne normalement fermée et ne s'ouvrant que lors d'une inspiration du patient).

Dans la variante d'exécution illustrée sur la fig. 2, sur laquelle les mêmes éléments que ceux de la fig. 1 sont affectés des mêmes numéros de référence, l'intérieur de la chambre 5 est mis à d'atmosphère au moyen d'un trou 17 percé dans une de ses parois, tandis que l'intérieur de la baudruche est relié à la canalisation 3 d'alimentation en oxygène, en aval de la vanne 2, par un conduit 18. Le fonctionnement de ce détecteur est l'inverse de celui décrit précédemment en référence à la fig. 1. A l'état de repos, c'est-à-dire tant qu'une pression atmosphérique règne dans la lunette d'oxygénothérapie 4, la baudruche 6 est gonflée et la lamelle formant écran 9 qu'elle porte, se trouve interposée entre l'émetteur 7a et le récepteur 7b du capteur optoélectronique 7. Par contre, au début de chaque phase d'inspiration, la faible dépression apparaissant dans la lunette d'oxygénothérapie 4 est transmise par le conduit 19 à l'intérieur de la baudruche 6 si bien que celle-ci s'écrase en escamotant l'écran 9. Le faisceau lumineux émis par l'émetteur 7a est alors capté par le récepteur 7b, ce qui se traduit par l'émission d'une impulsion de l'enclenchement de l'ouverture de la vanne 2, pendant une durée choisie à volonté, au moyen du circuit 16, comme dans le cas de l'exemple représenté sur la fig. 1.

La fig. 3 représente une variante d'exécution d'un détecteur utilisé avec une lunette d'oxygénothérapie 4 comportant un tuyau annexe 19 de prise de pression. Ce tuyau 19 est relié à l'intérieur de la chambre étanche 5, l'intérieur de la baudruche 6 étant mis à l'atmosphère comme dans le cas de la forme d'exécution représentée sur la fig. 1. Ainsi, pendant toute la phase inspiratoire, la dépression régnant dans le tuyau 19 est transmise à l'intérieur de la chambre étanche 5 si bien que la baudruche 6 se trouve être gonflée et qu'elle interpose la lamelle formant écran 9 entre l'émetteur 7a et le récepteur 7b du capteur optoélectronique 7. Le signal de sortie du capteur optoélectronique 7 est mis en forme par le circuit 13 et appliqué directement au circuit 15 de commande de l'électrovanne de manière à maintenir ouverte en permanence l'électrovanne 12 pendant toute la durée de la phase inspiratoire, c'est-à-dire tant qu'une dépression règne dans le tuyau 19.

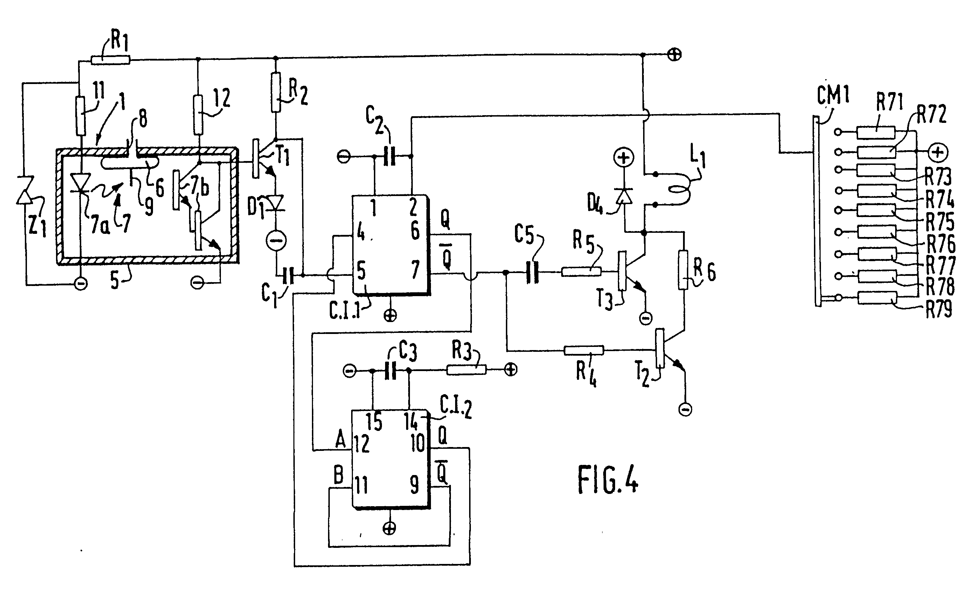

La fig. 4 décrit un schéma électronique explicitant le déclenchement d'une électrovanne à l'aide du dispositif selon l'invention. La chambre étanche 5, dont l'arrivée d'air en provenance du patient n'a pas été représentés sur la figure, comporte comme précédemment le capteur optoélectronique 7 constitué d'une part par la diode émettrice 7a et d'autre part par les deux phototransistors 7b montés en Darlington. Le collecteur commun aux deux phototransistors 7b est relié via la résistance 12 au plus de l'alimentation tandis que l'émetteur du transistor 7b de sortie du montage Darlington est relié au moins de l'alimentation. De même, le photodétecteur 7a est connecté par sa cathode au moins de l'alimentation et par son anode au plus de l'alimentation, par l'intermédiaire des résistances 11 et R1. Une diode zener Z1 est placée entre le moins et le point commun aux résistances 11 et R1 de manière à maintenir une tension constante entre ces deux points et ainsi minimiser les variations de tension aux bornes de la diode émettrice (ceci évite de voir la sensibilité varier en fonction de la tension de la batterie). La baudruche 6 et son écran 9 sont disposés comme précédemment dans l'enceinte étanche 5. Le point commun au collecteur des deux transistors 7b et relié à la base du transistor T1 dont le collecteur est relié au plus de l'alimentation par l'intermédiaire de la résistance R2. L'émetteur de T1 est relié à l'anode d'une diode T1 dont la cathode est au moins de l'alimentation. Un condensateur C1 de découplage est placé entre le collecteur de T1 et le moins de l'alimentation, le collecteur de T1 étant également relié à l'entrée 5 d'un circuit intégré de référence CD 4538. Ce circuit a été, pour des raisons de commodité, séparé en deux sous-ensembles CI1 et CI2 sur la figure. Chacun des deux circuits CI1 et CI2 est un circuit monostable. Les entrées 1 et 2 de CI1 sont reliées au condensateur C2. L'entrée 1 est également reliée au moins de l'alimentation, tandis que l'entrée 2 est reliée par l'intermédiaire du commutateur Cm1 à l'une des résistances R71, R72...R79 que l'on peut choisir de manière à obtenir une constante de temps Rc correspondant à la durée de l'impulsion .délivrée par le monostable CI1 sur sa sortie 6 (sortie Q). Celle-ci est reliée à l'entrée A (numéro 12) de CI2. L'entrée B (numéro 11) de CI2 est reliée à la sortie Q (numéro 9) de CI2, tandis que la sortie Q de CI2 (numéro 10) est reliée à l'entrée 4 de CI1. Entre les bornes 14 et 15 de CI2, est connecté un circuit C3, R3, permettant de fixer la constante de temps de ce monostable CI2. L'entrée 15 est reliée au moins de l'alimentation et l'entrée 14 est reliée au plus par l'intermédiaire de R3. La sortie Q de CI1 (numéro 7) est reliée d'une part à la base du transistor T3 par l'intermédiaire du condensateur C5 en série avec la résistance R5, et d'autre part à la base du transistor T2 par l'intermédiaire de la résistance R4. Les émetteurs de transistors T2 et T3 sont reliés au moins de l'alimentation, tandis que le collecteur de T2 est relié au collecteur de T3 par l'intermédiaire de la résistance R6. Le collecteur de T3 est relié à une extrémité de l'enroulement L1 de l'électrovanne V, dont l'autre extrémité est reliée au plus de l'alimentation. Une diode D4 relie également le collecteur de T3 au plus de l'alimentation.

Le fonctionnement de ce dispositif est le suivant. Lorsque le faisceau entre l'émetteur 7a et le récepteur 7b est coupé, au moins partiellement par la languette 9 (voir plus loins les différents modes de fonctionnement ainsi que les différents réglages possibles), le récepteur 7b se bloque ce qui engendre une remontée de la tension sur son collecteur et donc sur la base de T1 qui devient conducteur. Ceci entraîne une chute du potentiel sur le collecteur de T1 et la passage au niveau 0 de l'entrée 5 de CI1. Ceci engendre sur la sortie Q du monostable CI1 (numéro 6) le basculement au niveau «1» de celle-ci engendrant ainsi le déclenchement du monostable CI2. Sur la sortie Q de celui-ci (numéro 10) apparaît ainsi un signal dont la durée au niveau «1» sera fonction de la constante de temps R3 C3 et qui vient bloquer l'entrée 4 de CI1. Ceci permet d'éviter le redéclenchement de CI1 par un déplacement de la languette 9 de la baudruche 6 pendant un temps prédéterminé, évitant ainsi au circuit d'osciller de manière ininterrompue et provoquer des ouvertures et fermetures trop rapprochées de l'électrovanne EV. Sur la sortie Q de CI1 (numéro 7) apparaît donc le signal complémentaire au signal apparaîssant sur Q (niveau «0»). Le transistor T2 se bloque. L'électrovanne n'est plus alimentée (tension + sur le collecteur de T3). L'électrovanne EV s'ouvre, car il s'agit d'une électrovanne normalement ouverte en l'absence de courant pour des raisons de sécurité. A la fin de l'intervalle de temps prédéterminé par C2 et Cm1, Q remonte à l'état «1». Le signal engendre une impulsion sur la base du transistor T3 après passage dans le condensateur C5. Cette impulsion provoque la saturation du transistor T3 qui devient conducteur provoquant ainsi un passage intense de courant de l'enroulement L1 de l'électrovanne et donc la fermeture de celle-ci. Le maintien de la fermeture est assuré par T2 qui est conducteur pendant tout l'intervalle de temps pendant lequel Q est au niveau «1».

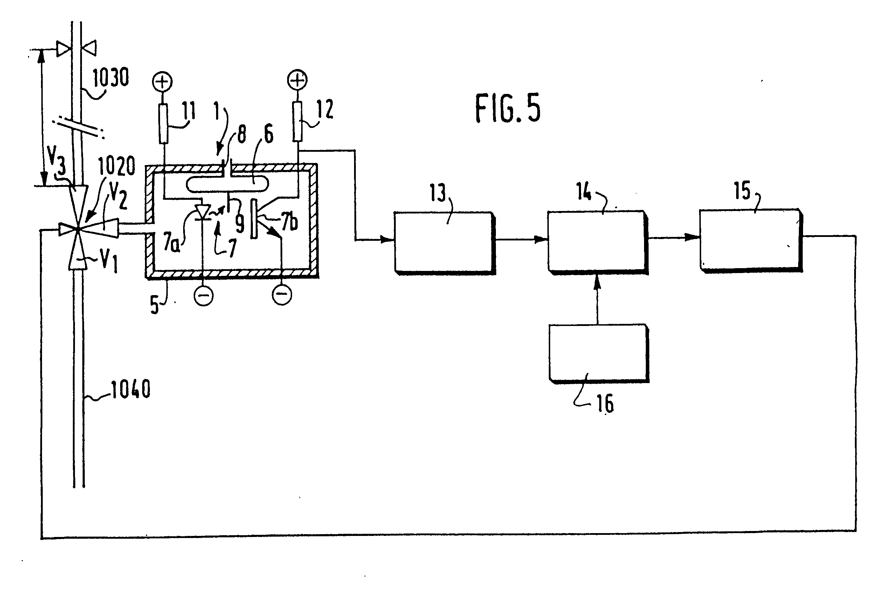

La fig. 5 illustre une variante de la fig. 2 avec une électrovanne à trois voies. Sur cette figure, les mêmes éléments que ceux des figures précédentes portent les mêmes références. Le seul élément modifié sur cette figure par rapport à la fig. 2 est la présence de l'électrovanne à trois voies 1020. Dans ce mode de fonctionnement, l'oxygène issu d'une source (non représentée sur la figure) est conduit au patient par l'intermédiaire de la canalisation 1030 puis l'électrovanne 1020 et enfin la canalisation 1040 qui débouche dans les narines du patient. L'électrovanne à trois voies 1020 évite l'envoi d'une pression dans le détecteur 1 en début d'inspiration. Lorsque le patient aspire via 1040, les voies V1 et V2 sont tout d'abord ouvertes et mises en communication l'une avec l'autre, ce qui provoque une légère dépression dans le capteur 1 engendrant ainsi via l'électronique 13, 14, 15 et 16 la commande de l'ouverture de l'électrovanne 1020, c'est-à-dire la mise en communication de la voie V3 avec la voie V1, sans communication de la voie V3 avec la voie V2. De cette manière, on évite de perturber le fonctionnement du capteur par l'envoi d'une pression en début d'inspiration.

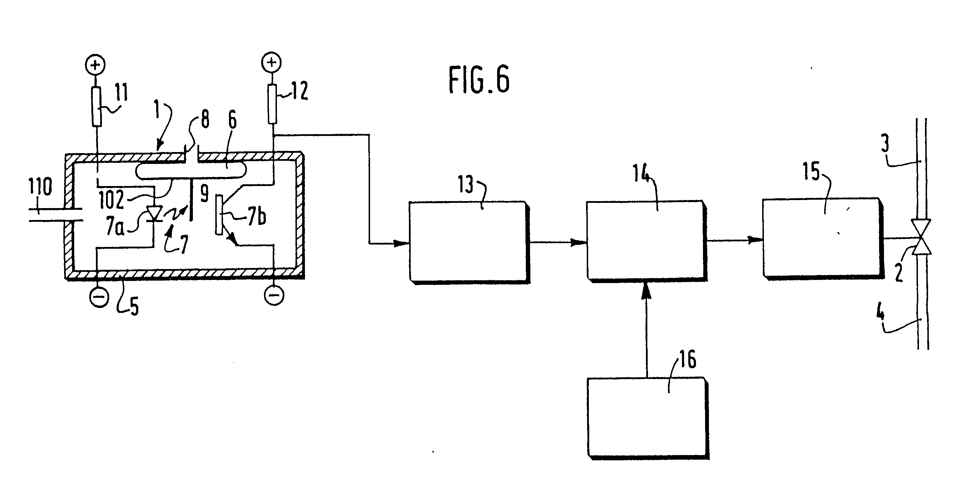

· La fig. 6 représente une variante de réalisation de l'invention avec un système de déclenchement en fin d'expiration. En effet, dans les dispositifs décrits ci-dessus, on utilise toujours l'effort inspiratoire du patient pour déclencher l'insufflation de l'oxygène ou du mélange gazeux. Dans certaines applications cependant, on a constaté que le temps de réponse de l'ensemble électronique et mécanique peut être assez long et supérieur à 100 et même 200 millisecondes. Ce temps de réponse dépend en effet en partie de l'effort inspiratoire du patient et sera d'autant plus long que cet effort est faible. Au commencement de l'inspiration, le premier gaz à pénétrer dans les alvéoles pulmonaires pendant l'inspiration est le gaz alvéolaire qui remplissait l'espace mort anatomique à la fin de l'expiration précédente. Il est très important que l'oxygène puisse arriver aux alvéoles dès le début de l'inspiration. Il est même souhaitable de remplacer le gaz alvéolaire de l'espace mort en fin d'expiration.

Afin d'améliorer le fonctionnement du capteur décrit ci-avant, le système selon la présente variante est tel que ce n'est plus l'inspiration qui déclenche la commande du système mais la fin de l'expiration du patient, quand il n'y a plus de débit expiratoire. La commande se fait donc pendant la période préinspiratoire ce qui, compte tenu des retards, permet une arrivée d'oxygène au tout début de la phase inspiratoire du patient ou même avant, ce qui est favorable puisque dans ce cas une partie de l'espace mort contient alors le mélange oxygène et gaz alvéolaire de fin d'expiration.

Pour utiliser le système selon cette variante, il suffit simplement de modifier le réglage du capteur 1. Dans ce cas, la position de repos de la lamelle 9, position dans laquelle cette lamelle coupe ou intercepte complètement ou partiellement le faisceau lumineux entre 7a et 7b, correspond à l'ouverture de la vanne d'alimentation en oxygène, ou plus exactement à la phase inspiratoire du patient. Dès le début de la phase expiratoire du patient, le gaz d'expiration qui parvient au détecteur 1 par l'intermédiaire de la canalisation 110, provoque une augmentation de pression à l'intérieur du détecteur 1, cette augmentation de pression engendrant une force sur la face inférieure 102 de la baudruche 6, ce qui déclenche la remontée vers le haut de la lamelle 9. Le faisceau lumineux entre 7a et 7b n'est plus intercepté par la lamelle 9. L'envoi d'un niveau «1» sur l'entrée 5 du monostable CI2 est sans effet. Dès que l'effort expiratoire du patient cesse, c'est-à-dire quand il n'y a plus de débit expiratoire, la lamelle descend et coupe le faisceau infra-rouge. Le point A devient positif (transistor 7b bloqué) ce qui déclenche de la manière explicitée sur la fig. 4 l'ouverture de l'électrovanne EV et l'insufflation d'oxygène pendant l'intervalle de temps prédéterminé. Bien entendu, l'utilisation d'un tel procédé décrit en regard de cette fig. 5 n'est pas limité à l'oxygénothérapie et à ce type de capteur. Elle peut s'appliquer dans tout domaine où il est préférable d'anticiper le déclenchement du cycle inspiratoire afin de délivrer le mélange gazeux en période préinspiratoire ou au tout début de l'inspiration du patient.

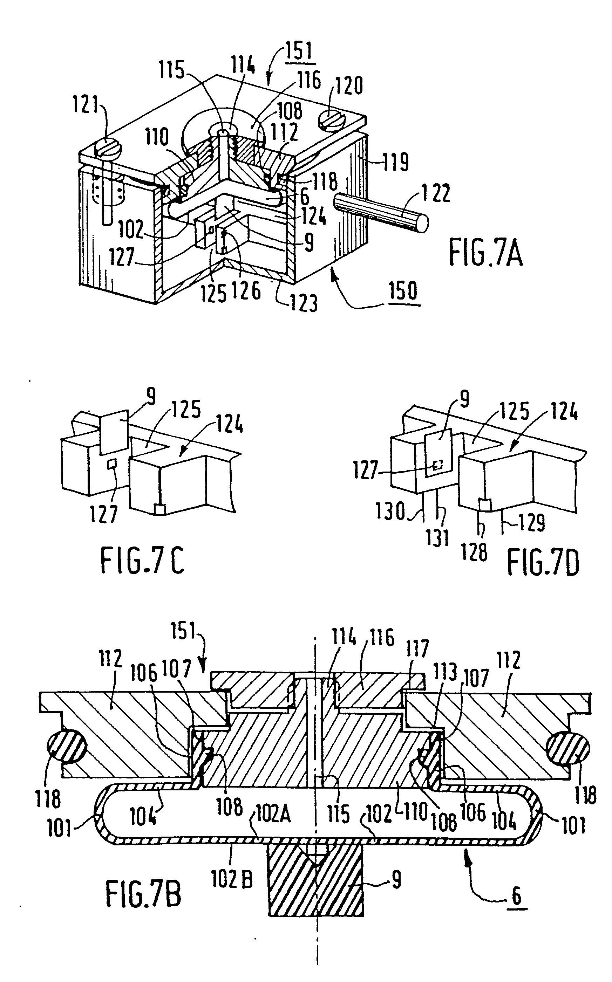

La fig. 7 représente une vue détaillée d'une réalisation d'un dispositif conforme à l'invention. La baudruche 6 comporte une surface inférieure 102 plane, à laquelle est fixée la lamelle 9 par exemple par collage. Cette face 102 se prolonge par deux surfaces latérales.

La fig. 7 représente différentes vues d'une réalisation d'un dispositif conforme à l'invention.

Sur la fig. 7A est représentée une vue en coupe partielle du dispositif selon l'invention. Celui-ci comporte un boîtier formé d'une embase 150 et d'un couvercle 151 solidaire l'un de l'autre par des vis 120 et 121 qui permettent le règlage en hauteur du couvercle 151 par rapport à l'embase 150, des ressorts (non représentés sur la figure) étant placés entre embase et couvercle pour appliquer une force vers le haut.

Dans l'embase 150 de paroi inférieure 123 et de parois latérales 119 est disposé un dispositif optoélectronique 124, comportant essentiellement une fente 125 avec un émetteur 127 et un récepteur 126 (ou vice-versa) disposés de part et d'autre de la fente. La couvercle 151, représenté en coupe sur la fig. 7B, comporte le couvercle proprement dit 112 dans lequel s'insère le support de baudruche 110 rendu solidaire de couvercle 112 par une pièce supérieure 116, celle-ci étant vissée autour du filetage 114 placé en saillie au centre du support 110. La pièce 116 repose par sa portée circulaire 117 sur l'ouverture cylindrique dans le couvercle 112, le support 110 venant en butée par sa partie supérieure contre la portée circulaire 113 interne du couvercle 112. La baudruche 6 est solidaire du support 110 par fixation de sa partie annulaire 1D6 dans la rainure 108. La partie 107 de la baudruche située au-dessus de 106 sert de joint d'étanchéité au niveau de la portée circulaire 113. La baudruche 6 se prolonge, au-delà de sa partie cylindrique verticale 106, 107, par une couronne circulaire horizontale 104. Celle-ci se prolonge, vue en coupe par une partie semi-circulaire 101, qui se prolonge par une surface plane circulaire 102. La languette 9 est solidaire de la surface 102, sensiblement en son centre. Le fonctionnement de ce dispositif est le suivant:

La baudruche 6 est en communication avec l'atmosphère extérieure par le canal 115 percé dans le support 110. L'air exerce donc une force sur la face 102A de la baudruche 6. La face extérieure 102B de la baudruche est au contact de l'air expiré et/ou inspiré par le patient qui parvient au détecteur 1 par la canalisation 122, la chambre étant maintenue étanche par le joint torique 118. Cet air «patient» exerce une force antagoniste sur la face 102B.

Lorsque la pression de l'air «patient» dans la chambre 1 est égale à la pression atmosphérique, la languette 9 ne coupe pas le faisceau lumineux entre 126 et 127 (dans l'exemple de la fig. 7C) lorsque le patient crée une dépression dans la chambre (inspiration) – fig. 7D, la languette coupe tout ou partie du faisceau (suivant règlage), la force exercée par l'air ambiant sur 102A étant supérieure à celle exercée par l'air «patient» 102B et déformant la membrane 102 (celle-ci devient convexe). Inversement, au cours de l'expiration, l'air «patient» dans la chambre 1 est à pression supérieure à l'air ambiant et la membrane 102 est repoussée vers le haut (elle prend une forme concave), et la languette ne coupe plus le faisceau.

Le fait qu'il existe trois positions possibles pour la membrane 102 (plane, concave, convexe) peut être utilisé pour un fonctionnement décrit en regard de la fig. 6. Lorsque la membrane 102 est au repos (plane), la languette coupe le faisceau – lorsque le patient inspire, la languette ne coupe plus totalement (ou partiellement) le faisceau. Dès la fin de l'inspiration, la pression dans la chambre augmente, la languette coupe alors le faisceau partiellement (ou totalement selon le règlage du circuit électronique) et l'on déclenche le circuit électronique de la fig. 4 de manière à ouvrir l'électrovanne d'oxygène. L'émetteur 7a et le récepteur 7b sont respectivement connectés électriquement (selon fig. 4) par les connexions électriques représentées par 130, 131 et 128, 129.

Bien que dans la description qui précède on ait considéré principalement l'application du détecteur de dépression à l'oxygénothérapie, il va de soi qu'il peut être utilisé dans n'importe quel domaine exigeant la détection de dépressions inférieurs à 0,1 millibars et il peut être également utilisé chaque fois qu'il est nécessaire de détecter de faibles pressions différentielles, c'est-à-dire de faibles variations de pression par rapport à une pression de référence.

**Revendications**

1. Appareil pour le traitement des déficiences respiratoires d'un patient par la fourniture d'un fluide de respiration à partir d'une source de fluide de respiration afin de compléter la fourniture d'oxygène inspiré par le patient dans l'air ambiant, cet appareil comportant un premier conduit (3, 4) reliant la source de fluide de respiration à la bouche ou aux narines du patient, une vanne (2) dont l'ouverture et la fermeture peuvent être commandées électriquement étant placée dans ledit premier conduit (3, 4) de manière à contrôler le débit de fluide de respiration délivré au patient et des moyens pour commander l'ouverture ou la fermeture de la vanne en liaison avec les phases d'inspiration et d'expiration du patient, caractérisé en ce que les moyens pour commander l'ouverture ou la fermeture de la vanne sont constitués d'une chambre (5) dans laquelle est placé un réservoir (6) sous forme d'une baudruche souple, ce réservoir (6) ayant une ouverture (115) débouchant à l'air ambiant à travers une cloison (112) de la chambre (5), d'un écran (9) solidaire d'une paroi mobile (102) dudit réservoir (6), d'un dispositif optoélectronique (7) comportant un émetteur (7a) et un récepteur (7b) d'un faisceau lumineux, d'un circuit électronique de commande (13, 14, 15, 16) connectée, d'une part, au récepteur (7b) et, d'autre part, à la vanne (2), ainsi que d'un second conduit (10, 19) reliant la chambre (5) à la bouche ou aux narines du patient, la paroi mobile (102) dudit réservoir étant soumise d'un côté à la pression de l'air ambiant et de l'autre à la pression de l'air dans la chambre (5) venant de/ou allant vers le patient par l'intermédiaire du second conduit (10, 19), la différence de pression de part et d'autre de la paroi mobile (102) du réservoir engendrant un déplacement de l'écran (9) en direction du gaz de plus faible pression, de sorte que l'écran (9) vient couper au moins partiellement ou cesse de couper totalement le faisceau lumineux entre émetteur (7a) et récepteur (7b), ledit récepteur (7b) engendrant des signaux de déclenchement du circuit électronique de commande (13, 14, 15, 16) qui provoquent l'ouverture ou la fermeture de la vanne (2).

2. Appareil pour le traitement des déficiences respiratoires d'un patient par la fourniture d'un fluide de respiration à partir d'une source de fluide de respiration afin de compléter la fourniture d'oxygène inspiré par le patient dans l'air ambiant, cet appareil comportant un premier conduit (3, 4) reliant la source de fluide de respiration à la bouche ou aux narines du patient, une vanne (2) dont l'ouverture de la fermeture peuvent être commandées électriquement étant placée dans ledit premier conduit (3, 4) de manière à contrôler le débit de fluide de respiration délivré au patient et des moyens pour commander l'ouverture ou la fermeture de la vanne en liaison avec les phases d'inspiration et d'expiration du patient, caractérisé en ce que les moyens pour commander l'ouverture ou la fermeture de la vanne sont constitués d'une chambre (5) dans laquelle est placé un réservoir (6) sous forme d'une baudruche souple, d'un écran (9) solidaire d'une paroi mobile (102) dudit réservoir (6), d'un dispositif optoélectronique (7) comportant un émetteur (7a) et un récepteur (7b) d'un faisceau lumineux, d'un circuit électronique de commande (13, 14, 15, 16) connecté, d'une part, au récepteur (7b) et, d'autre part, à la vanne (2), ainsi que d'un second conduit (18) reliant l'intérieur du réservoir (6) au premier conduit (3, 4) en aval de la vanne (2), l'intérieur de la chambre (5) étant mis à l'atmosphère au moyen d'un trou (17) percé dans une des parois de cette chambre, de sorte que la paroi mobile (102) dudit réservoir soit soumise du côté de la chambre à la pression de l'air ambiant et de l'autre à la pression de l'air venant de/ou allant vers le patient par l'intermédiaire du second conduit (18), la différence de pression de part et d'autre de la paroi mobile (102) du réservoir engendrant un déplacement de l'écran (9) en direction du gaz de plus faible pression, de sorte que l'écran (9) vient couper au moins partiellement ou cesse de couper totalement le faisceau lumineux entre émetteur (7a) et récepteur (7b), ledit récepteur (7b) engendrant des signaux de déclenchement du circuit électronique de commande (13, 14, 15, 16) qui provoquent l'ouverture ou la fermeture de la vanne (2).

3. Appareil selon la revendication 2, caractérisé en ce que l'écran (9) coupe au moins partiellement le faisceau lumineux lorsque la pression est identique de part et d'autre de la paroi mobile (102), et en ce que l'écran (9) ne coupe plus que partiellement le faisceau lumineux lorsque la pression du gaz dans le réservoir (6) est inférieure à celle du gaz dans la chambre (5).

4. Appareil selon la revendication 1, caractérisé en ce que l'écran (9) ne coupe pas ou ne coupe que partiellement le faisceau lumineux lorsque la pression est identique de part et d'autre de la paroi mobile (102) et en ce que l'écran (9) coupe au moins partiellement ou coupe totalement le faisceau lumineux lorsque la pression du gaz dans le réservoir (6) est supérieure à celle du gaz dans la chambre (5).

5. Appareil selon l'une des revendications 1 à 3, caractérisé en ce que le second conduit (10, 19) est relié au premier (3, 4) entre la vanne (2) et la bouche ou les narines du patient.

6. Appareil selon l'une des revendications 1 ou 4, caractérisé en ce que l'écran (9) a la forme d'une lamelle plate directement fixée sur la paroi mobile (102) du réservoir (6) qui est placé au-dessus de l'émetteur (7a) et du récepteur (7b).

7. Appareil selon l'une des revendications 1 à 6, caractérisé en ce que la paroi mobile (102) du réservoir (6) a une souplesse telle qu'une variation de pression d'un millimètre d'eau de part et d'autre entraîne un déplacement de l'écran (9) d'au moins 0,05 mm.

8. Appareil selon l'une des revendications 1 à 7, caractérisé en ce que la chambre (5) comporte des moyens (120, 121) pour régler en hauteur la position de l'écran (9) par rapport au faisceau lumineux du dispositif optoélectronique (7).

9. Appareil selon la revendication 3, caractérisé en ce que l'on règle au repos la position de l'écran (9) par rapport au faisceau lumineux de manière à ce que ledit écran (9) coupe partiellement ou totalement le faisceau lumineux lors de l'expiration du patient, mais ne coupe plus ou plus que partiellement ledit faisceau à la fin de la phase d'expiration du patient, déclenchant l'ouverture de la vanne (2) dès la fin de la phase d'expiration et au plus tard au débit de la phase d'inspiration du patient.

10. Appareil selon la revendication 3, caractérisé en ce que l'on règle au repos la position de l'écran (9) par rapport au faisceau lumineux de manière à ce que ledit écran (9) ne coupe pas ou ne coupe que partiellement le faisceau lumineux lors de l'expiration du patient, mais coupe partiellement ou totalement le faisceau lumineux dès le début de la phase d'inspiration du patient, déclenchant l'ouverture de la vanne (2) au début de la phase d'inspiration du patient.

11. Appareil selon la revendication 2, caractérisé en ce que le second conduit (18) est relié à l'ouverture (115) du réservoir (6) tandis que la chambre (5) comporte une seconde ouverture (17) débouchant dans l'air ambiant.

12. Dispositif de commande d'utilisable dans un appareil selon la revendication 1, caractérisé en ce qu'il comporte une chambre (5) dans laquelle est placé un réservoir (6) sous forme d'une baudruche souple, ce réservoir (6) ayant une ouverture (115) débouchant à l'air ambiant à travers un trou (8) dans une première paroi (112) de ladite chambre, ce réservoir (6) comportant une paroi souple mobile (102), sur laquelle est fixé un écran (9), ladite paroi (102) étant placée au-dessus d'un dispositif optoélectronique (7), disposé sur une seconde paroi (123) de la chambre (5), opposée à la première (112), et comportant un dispositif émetteur (7a) d'un faisceau lumineux ainsi qu'un dispositif récepteur (7b) dudit faisceau lumineux, l'écran mobile (9) pouvant se déplacer entre l'émetteur (7a) et le récepteur (7b) de manière à interrompre le faisceau lumineux, une ouverture (17) étant ménagée dans une troisième paroi (119) de ladite chambre (5), cette ouverture (17) étant destinée à recevoir un conduit (122) en liaison avec la bouche ou les marines du patient.

**Patentansprüche**

1. Vorrichtung zum Behandeln der unzureichenden Atmung eines Patienten durch Zuführen eines Atemfluids aus einer Atemfluidquelle zur Ergänzung der Versorgung der Umgebungsluft mit von dem Patienten eingeatmete Sauerstoff, umfassend eine erste Leitung (3, 4), die die Atemfluidquelle mit dem Munde oder den Nasenlöchern des Patienten verbindet, ein Ventil (2), dessen Öffnen und Schließen elektrisch gesteuert werden kann und das so in der genannten ersten Leitung (3, 4) angebracht ist, daß die Menge des dem Patienten zugeführten Atemfluids gesteuert wird, sowie Mittel zum Steuern des Öffnens oder des Schließens des Ventils im Zusammenhang mit den Einatmungs- und Ausatmungsphasen des Patienten, dadurch gekennzeichnet, daß die Mittel zum Steuern des Öffnens oder des Schließens des Ventils gebildet sind von einer Kammer (5), in der ein Behälter (6) aus einer nachgiebigen Goldschlägerhaut angeordnet ist, welcher Behälter (6) eine Öffnung (115) aufweist, die durch eine Trennwand (112) der Kammer (5) hindurch in die Umgebungsluft mündet, ferner von einer mit einer beweglichen Wand (102) des genannten Behälters (6) fest verbundenen Abschirmung (9), einer optoelektronischen Vorrichtung (7) mit einem Sender (7a) und einem Empfänger (7b) für ein Strahlenbündel, einer elektronischen Steuerschaltung (13, 14, 15, 16), die einerseits mit dem Empfänger (7b) und andererseits mit dem Ventil (2) verbunden ist, sowie einer zweiten Leitung (10, 19), die die Kammer (5) mit dem Munde oder den Nasenlöchern des Patienten verbindet, daß die bewegliche Wand (102) des genannten Behälters auf der einen Seite dem Druck der Umgebungsluft und auf der anderen Seite dem Druck der in der Kammer (5) befindlichen Luft, die über die zweite Leitung (10, 19) von dem Patienten herkommt oder dem Patienten zufließt, ausgesetzt ist, wobei der Unterschied der Drücke auf den beiden Seiten der beweglichen Wand (102) des Behälters eine Bewegung der Abschirmung (9) in Richtung auf das Gas mit niedrigerem Druck herbeiführt, so daß die Abschirmung (9) das Strahlenbündel zwischen Sender (7a) und Empfänger (7b) wenigstens teilweise unterbricht oder nicht mehr vollständig unterbricht, wodurch der genannte Empfänger (7b) Signale zum Auslösen der elektronischen Steuerschaltung (13, 14, 15, 16) erzeugt, die das Öffnen oder das Schließen des Ventils (2) herbeiführen.

2. Vorrichtung zum Behandeln der unzureichenden Atmung eines Patienten durch Zuführen eines Atemfluids aus einer Atemfluidquelle zur Ergänzung der Versorgung der Umgebungsluft mit dem von dem Patienten eingeatmeten Sauerstoff, umfassend eine erste Leitung (3, 4), die die Atemfluidquelle mit dem Munde oder den Nasenlöchern des Patienten verbindet, ein Ventil (2), dessen Öffnen und Schließen elektrisch gesteuert werden kann und das so in der genannten ersten Leitung (3, 4) angebracht ist, daß die dem Patienten zugeführte Menge Atemfluid gesteuert wird, sowie Mittel zum Steuern des Öffnens oder des Schließens des Ventils im Zusammenhang mit den Einatmungs- und Ausatmungsphasen des Patienten, dadurch gekennzeichnet, daß die Mittel zum Steuern des Öffnens oder des Schließens des Ventils gebildet sind von einer Kammer (5), in der ein Behälter (6) aus einer nachgiebigen Goldschlägerhaut angeordnet ist, einer mit einer beweglichen Wand (102) des genannten Behälters (6) fest verbundenen Abschirmung (9), einer optoelektronischen Vorrichtung (7) mit einem Sender (7a) und einem Empfänger (7b) für ein Strahlenbündel, einer elektronischen Steuerschaltung (13, 14, 15, 16), die einerseits mit dem Empfänger (7b) und andererseits mit dem Ventil (2) verbunden ist, sowie einer zweiten Leitung (18), die den Innenraum des Behälters (6) mit der ersten Leitung (3, 4) stromab von dem Ventil (2) verbindet, und daß der Innenraum der Kammer (5) mit der Umgebungsluft durch ein in eine der Wände dieser Kammer gebohrtes Loch (17) verbunden ist, so daß die bewegliche Wand (102) des genannten Behälters auf der Kammerseite dem Druck der Umgebungsluft ausgesetzt ist und auf der anderen Seite dem Druck der Luft, die über die zweite Leitung (18) von dem Patienten herkommt oder dem Patienten zufließt, wobei der Unterschied der Drücke auf den beiden Seiten der beweglichen Wand (102) des Behälters eine Bewegung der Abschirmung (9) in Richtung auf das Gas mit niedrigerem Druck herbeiführt, so daß die Abschirmung (9) das Strahlenbündel zwischen Sender (7a) und Empfänger (7b) wenigstens teilweise unterbricht oder nicht mehr vollständig unterbricht, wodurch der genannte Empfänger (7b) Signale zum Auslösen der elektronischen Steuerschaltung (13, 14, 15, 16) erzeugt, die das Öffnen oder das Schließen des Ventils (2) herbeiführen.

3. Vorrichtung nach Anspruch 2, dadurch gekennzeichnet, daß die Abschirmung (9) das Strahlenbündel wenigstens teilweise unterbricht, wenn der Druck auf beiden Seiten der beweglichen Wand (102) gleich hoch ist, und daß die Abschirmung (9) das Strahlenbündel nur noch teilweise unterbricht, wenn der Druck des Gases in dem Behälter (6) niedriger ist als der Druck des Gases in der Kammer (5).

4. Vorrichtung nach Anspruch 1, dadurch gekennzeichnet, daß die Abschirmung (9) das Strahlenbündel nicht oder nur teilweise unterbricht, wenn der Druck auf den beiden Seiten der beweglichen Wand (102) gleich hoch ist, und daß die Abschirmung (9) das Strahlenbündel wenigstens teilweise oder vollständig unterbricht, wenn der Druck des Gases in dem Behälter (6) höher ist als der Druck des Gases in der Kammer (5).

5. Vorrichtung nach einem der Ansprüche 1 bis 3, dadurch gekennzeichnet, daß die zweite Leitung (10, 19) zwischen dem Ventil (2) und dem Munde oder den Nasenlöchern des Patienten an die erste Leitung (3, 4) angeschlossen ist.

6. Vorrichtung nach Anspruch 1 oder 4, dadurch gekennzeichnet, daß die Abschirmung (9) die Form einer ebenen Lamelle hat, die unmittelbar an der beweglichen Wand (102) des Behälters (6) befestigt ist, welche sich oberhalb des Senders (7a) und des Empfängers (7b) befindet.

7. Vorrichtung nach einem der Ansprüche 1 bis 6, dadurch gekennzeichnet, daß die bewegliche Wand (102) des Behälters (6) derart nachgiebig ist, daß eine Veränderung des Drucks von 1 mm Wassersäule zu beiden Seiten zu einer Bewegung der Abschirmung (9) von mindestens 0,05 mm führt.

8. Vorrichtung nach einem der Ansprüche 1 bis 7, dadurch gekennzeichnet, daß die Kammer (5) Mittel (120, 121) zur Einstellung der Höhe der Lage der Abschirmung (9) gegenüber dem Strahlenbündel der optoelektronischen Vorrichtung (7) aufweist.

9. Vorrichtung nach Anspruch 3, dadurch gekennzeichnet, daß für den Ruhezustand die Lage der Abschirmung (9) gegenüber dem Strahlenbündel so eingestellt wird, daß die genannte Abschirmung (9) das Strahlenbündel bei der Ausatmung des Patienten teilweise oder vollständig unterbricht, aber das genannte Strahlenbündel am Ende der Ausatmungsphase des Patienten nicht mehr oder nur noch teilweise unterbricht und dadurch das Öffnen des Ventils (2) schon am Ende der Ausatmungsphase auslöst und spätestens am Beginn der Einatmungsphase des Patienten.

10. Vorrichtung nach Anspruch 3, dadurch gekennzeichnet, daß für den Ruhezustand die Lage der Abschirmung (9) gegenüber dem Strahlenbündel so eingestellt wird, daß die genannte Abschirmung (9) das Strahlenbündel bei der Ausatmung des Patienten nicht oder nur teilweise unterbricht, aber schon am Beginn der Einatmungsphase des Patienten das Strahlenbündel teilweise oder vollständig unterbricht, wodurch das Öffnen des Ventils (2) zu Beginn der Einatmungsphase des Patienten ausgelöst wird.

11. Vorrichtung nach Anspruch 2, dadurch gekennzeichnet, daß die zweite Leitung (18) mit der Öffnung (115) des Behälters (6) verbunden ist, während die Kammer (5) eine zweite Öffnung (17) aufweist, die in die Umgebungsluft mündet.

12. Steuervorrichtung, die bei einer Vorrichtung nach Anspruch 1 einsetzbar ist, dadurch gekennzeichnet, daß sie eine Kammer (5) aufweist, in welcher ein Behälter (6) aus einer nachgiebigen Goldschlägerhaut angeordnet ist, welcher Behälter (6) eine Öffnung (115) besitzt, die durch ein Loch (8) in einer ersten Wand (112) der genannten Kammer zur Umgebungsluft ausmündet, daß der Behälter (6) eine bewegliche nachgiebige Wand (102) aufweist, an der eine Abschirmung (9) befestigt ist, welche genannte Wand (102) oberhalb einer optoelektronischen Vorrichtung (7) angeordnet ist, die sich an einer der ersten Wand (112) gegenüberstehenden zweiten Wand (123) der Kammer (5) befindet und eine Vorrichtung (7a) zum Aussenden eines Strahlenbündels sowie eine Vorrichtung (7b) zum Empfangen des genannten Strahlenbündels umfaßt, wobei die bewegliche Abschirmung (9) sich zwischen dem Sender (7a) und dem Empfänger (7b) so bewegen kann, daß sie das Strahlenbündel unterbricht, und daß eine Öffnung (17) in einer dritten Wand (119) der genannten Kammer (5) vorgesehen ist, welche Öffnung (17) zur Aufnahme einer zu dem Munde oder den Nasenlöchern des Patienten führenden Leitung (122) bestimmt ist.

**Claims**

1. Apparatus for treating respiratory deficiencies of a patient by supplying a respiratory fluid from a source of respiratory fluid to complement the supply of oxygen breathed in by the patient from the ambient air, this apparatus comprising a first conduit (3, 4) connecting the respiratory fluid source to the mouth or the nostrils of the patient, a valve (2) the opening and closing of which may be operated electrically, being arranged in the said first conduit (3, 4) in such a way as to control the flow rate of respiratory fluid supplied to the patient and means for operating the opening or closing of the valve in conjunction with the inhalation and exhalation stages of the patient, characterised in that the means for operating the opening or closing of the valve are constituted by a · chamber (5) wherein is situated a reservoir (6) in the form of a flexible bag (6), this reservoir (6) having an aperture (115) opening into the ambient air through a partition (112) of the chamber (5), a screen (9) integral with a movable wall (102) of the said reservoir (6), an opto-electronic device (7) comprising an emitter (7a) and a receiver (7b) of a light beam, an electronic control circuit (13, 14, 15, 16) connected on the one hand to the receiver (7b) and on the other hand to the valve (2), as well as a second conduit (10, 19) connecting the chamber (5) to the patient's mouth or nostrils, the movable wall (102) of the reservoir being exposed to the ambient air pressure on one side and· to the pressure of the air in the chamber on the other side, moving towards or away from the patient by way of the second conduit (10, 19), the pressure difference between the two sides of the movable wall (102) of the reservoir giving rise to a displacement of the screen (9) in the direction of the gas of lower pressure, so that the screen (9) at least partially interrupts or ceases to interrupt totally the light beam between the emitter (7a) and the receiver (7b), the said receiver (7b) generating signals for triggering the electronic control circuit (13, 14, 15, 16) which cause the opening or closure of the valve (2).

2. Apparatus for treating respiratory deficiencies of a patient by supplying a respiratory fluid from a source of respiratory fluid to complement the supply of oxygen breathed in by the patient from the ambient air, this apparatus comprising a first conduit (3, 4) connecting the respiratory fluid source to the patient's mouth or nostrils, a valve (2) the opening and closing of which may be operated electrically being arranged in the said first conduit (3, 4) in such a way as to control the flow rate of respiratory fluid supplied to the patient and means for operating the opening or closing of the valve in conjunction with the inhalation and exhalation stages of the patient, characterised in that the means for operating the opening or closing of the valve comprise a chamber (5) wherein is situated a reservoir (6) in the form of a flexible bag (6), a screen (9) integral with a movable wall (102) of the said container (6), an opto-electronic device (7) comprising a emitter (7a) and a receiver (7b) of a light beam, an electronic control circuit (13, 14, 15, 16) connected on the one hand to the receiver (7b) and on the other hand to the valve (2), as well as a second conduit (18) connecting the inside of the reservoir (6) to the first conduit (3, 4) downstream of the valve (2), the inside of the chamber (5) being vented to the atmosphere by means of a hole (17) made in on of the walls of this chamber, so that the movable wall (102) of the said reservoir is exposed on the chamber side to the ambient air pressure and on the other side to the pressure of the air flowing from or towards the patient by way of the second conduit (18), the pressure difference between the two sides of the movable wall (102) of the reservoir giving rise to a displacement of the screen (9) in the direction of the gas of lower pressure, so that the screen (9) at least partially interrupts or ceases to interrupt totally the light beam between the emitter (7a) and the receiver (7b), the said receiver (7b) generating signals for triggering the electronic control circuit (13, 14, 15, 16) which cause the opening or closing of the valve (2).

3. Apparatus according to claim 2, characterised in that the screen (9) at least partially interrupts the light beam when the pressure is identical on both sides of the movable wall (102) and in that the screen (9) interrupts the light beam no more than partially when the pressure of the gas in the reservoir (6) is lower than that of the gas in the chamber (5).

4. Apparatus according to claim 1, characterised in that the screen (9) does not interrupt or only partially interrupts the light beam when the pressure is identical on both sides of the movable wall (102) and in that the screen (9) at least partially or completely interrupts the light beam when the pressure of gas in the reservoir (6) is higher than that of the gas in the chamber (5).

5. Apparatus according to one of the claims 1 to 3, characterised in that the second conduit (10, 19) is connected to the first (3, 4) between the valve (2) and the patient's mouth or nostrils.

6. Apparatus according to one of th claims 1 or 4, characterised in that the screen (9) has the form of a flat sheet secured directly on the movable wall (102) of the reservoir (6) which is situated above the emitter (7a) and the receiver (7b).

7. Apparatus according to one of the claims 1 to 6, characterised in that the movable wall (102) of the reservoir (6) has a flexibility such that a pressure variation of one millimetre of water one way or the other causes a displacement of the screen (9) by at least 0.05 mm.

8. Apparatus according to one of the claim 1 to 7, characterised in that the chamber (5) comprises means (120, 121) of vertically controlling the position of the screen (9) relative to the light beam of the opto-electronic device (7).

9. Apparatus according to claim 3, characterised in that the position of the screen (9) relative to the light beam is adjusted in the idle state in such a way that the said screen (9) partly or wholly interrupts the light beam during the patient's exhalation but interrupts the said beam only partly or not at all at the end of the patient's exhalation stage, triggering the opening of the valve (2) from the exhalation stage and no later than the beginning of the patient's inhalation stage.

10. Apparatus according to claim 3, characterised in that the position of the screen (9) relative to the light beam is adjusted in the idle state in such a way that the said screen (9) interrupts the light beam only partly or not at all during the patient's exhalation, but partly or wholly interrupts the light beam from the beginning of the patient's inhalation stage, triggering the opening of the valve (2) at the beginning of the patient's inhalation stage.

11. An apparatus according to claim 2, characterised in that the second conduit (18) is connected to the aperture (115) of the reservoir (6) whereas the chamber (5) comprises a second aperture (17) opening into the ambient air.

12. A control device usable in an apparatus according to claim 1, characterised in that it comprises a chamber (5) in which is situated a reservoir (6) in the form of a flexible bag, this reservoir (6) having an aperture (115) opening to the ambient air through a hole (8) in a first wall (112) of the said chamber this reservoir (6) comprising a movable flexible wall (102) on which is secured a screen (9), the said wall (102) being situated above an opto-electronic device (7) arranged on a second wall (123) of the chamber (5), opposed to the first (112), and comprising a light beam emitter device (7a) as well as a device (7b) for reception of the said light beam, the movable screen (9) being displaceable between the emitter (7a) and the receiver (7b) in such a way as to interrupt the light beam, an opening (17) being made in a third wall (119) of the said chamber (5), this opening (17) being intended to receive a conduit (122) in communication with the patient's mouth or nostrils.

FIG.1

FIG.2

FIG.3

FIG.4

FIG.5

EP 0183593 B1

FIG.6

FIG.7A

FIG.7C

FIG.7D

FIG.7B